# EUROPEAN PATENT SPECIFICATION

(11) **EP 0 386 057 B1**
(45) Date of publication and mention of the grant of the patent: **23.03.1994**
(21) Application number: 88909563.4
(22) Date of filing: 10.11.1988
(51) Int. Cl.: C12M 1/40

(54) **BIOELECTROCHEMICAL REACTIONS IN ORGANIC SOLVENTS**
BIOELEKTROCHEMISCHE REAKTIONEN IN ORGANISCHEN LÖSUNGSMITTELN
REACTIONS BIO-ELECTROCHIMIQUES DANS DES SOLVANTS ORGANIQUES

(30) Priority: 13.11.1987 GB 8726570; 21.04.1988 GB 8809485
(43) Date of publication of application: 12.09.1990
(73) Proprietor: Cranfield Biotechnology Limited, Cranfield, Bedford MK43 OAL (GB)
(72) Inventor: TURNER, Anthony, Peter, Francis, Cranfield Bedfordshire MK43 0DS (GB); BEST, David, John, Corbridge Northumberland NE45 5LX (GB); HALL, Geoffrey, Frank, Cranfield Bedfordshire MK43 0DJ (GB)
(74) Representative: Stuart, Ian Alexander
(86) International application number: GB8800970
(87) International publication number: WO8904364

(56) References cited:
- EP-A- 0 214 336
- US-A- 4 145 255
- ENZYME & MICROBIAL TECHNOLOGY, vol. 10, no. 9, September 1988, Butterworth Publishers; G.F. HALL et al., pp. 543-546#
- JOURNAL OF THE AMERICAN CHEMICAL SOCIETY, vol. 107, 1985, American Chemical Society, Washington, DC (US); R.Z. KAZANDIJAN et al., pp. 5448-5450#
- CHEMICAL ABSTRACTS, vol. 88, 1978, Columbus, OH (US); L. MACHOLAN et al., p. 297, no. 125937j#
- MOLECULAR BIOLOGY & BIOTECHNOLOGY, vol. 298, 1985, Special Pub. 54, Royal Soc. Chem., Walker & Gingold (eds.); pp. 314-317#
- BIOSENSORS, vol. 1, 1985; pp. 6-7#

## Description

### TECHNICAL FIELD

The present invention relates to bioelectrochemical reactions carried out in non-aqueous or microaqueous solvents. A microaqueous solvent is one formed by the addition of small quantities of water to a non-aqueous solvent (Yamane et al, 1988) and as used herein the term includes water-immiscible solvents which are saturated with water.

In particular, the present invention relates to a method of carrying out a bioelectrochemical reaction in a non-aqueous or microaqueous solution, the use of such a method for the determination of an analyte, and to an electrochemical cell and an enzyme electrode for carrying out such methods.

### BACKGROUND ART

It is well known to employ an enzyme electrode in order to perform and monitor a bioelectrochemical reaction in aqueous solution. For example, an enzyme electrode involving glucose oxidase may be used to effect the oxidation of glucose, and consequently to monitor the concentration of glucose in aqueous solution (see eg Turner et al, 1985). In such electrodes the enzyme is, conventionally, immobilised on the electrode by means of covalent bonding, and electron transfer between the redox centre of the enzyme and the electrode surface may be effected by means of a mediator molecule such as ferrocene (Cass et al 1984)

The use of enzyme electrodes in aqueous solutions enables the concentration of chemical substances in samples to be determined without extensive preparation. The enzyme provides the specificity of a biochemical reaction and the electrode monitors the extent or progress of the reaction in a sensitive manner (Turner et al, 1987).

EP-A-0 214 336 discloses an enzyme electrode for determining alcohol in water-immiscible solvents. An enzyme is immobilised on an electrode so as to be present in an aqueous phase. Alcohol must partition into the aqueous phase in order to interact with the enzyme.

However, the methods used to date suffer from several disadvantages. For example, the method is limited to the determination of species which are relatively water soluble, the electrode material must be one which is stable and operable in an aqueous solvent, and the method is not appropriate for use at elevated temperatures because of poor thermal stability of many enzymes in aqueous enviroments.

The present inventors have found that it is possible to carry out bioelectrochemical reactions in organic or microaqueous solvents. Although enzyme reactions in organic and microaqueous solvents have been reported (Klibanov, 1986; Halling, 1987; Kazandijan et al, 1985) the possibility of employing enzyme electrodes in organic electrochemistry has not, previously, been explored.

### DISCLOSURE OF THE INVENTION

According to one aspect of the present invention there is provided a method for carrying out a bioelectrochemical reaction in a non-aqueous or microaqueous solvent, the method comprising contacting a non-aqueous or microaqueous solution of a substrate for an enzyme with an electrode at which said enzyme is retained and allowing the substrate to undergo reaction at the electrode under the influence of said enzyme. One possibility is that the enzyme catalyses the conversion of the substrate into a product which then undergoes an electrochemical reaction directly at the electrode. An alternative is that the enzyme is one which can effect oxidation or reduction of the substrate, possibly with the intervention of a mediator, and is thus involved in the transfer of electrons between the substrate and the electrode. The enzyme may be present as a component of a whole cell, cell membrane, or organelle, or as a purified substance.

By carrying out the bioelectrochemical reaction in non-aqueous or microaqueous solvent the enzyme specificity may be made different from that in aqueous solution and the possibility exists of selecting particular specificities by making an appropriate choice of non-aqueous solvent. Furthermore, the solvent may be chosen so as to stabilise the enzyme substrate or product and hence enable the observation of otherwise difficult electrochemistry. Since thermal stability of enzymes is often enhanced in non-aqueous solvents reactions may also be carried out at elevated temperatures.

The method may be employed in the determination of an analyte in non-aqueous or microaqueous solution by including a non-aqueous or microaqueous solution to be analysed for said analyte in an electrochemical cell, said cell having an electrode at which an enzyme is retained; and by measuring an electrical response of said cell.

There are various possible analytes which might be detected. Principally, these are enzyme substrates or cofactors for said enzyme; redox species capable of mediating electron transfer with the enzyme of the electrode; or substances convertible to any of these. Analytes of low water solubility may now be determined for example by concentrating analyte from a large volume of water into a smaller quantity of non-aqueous solvent, for example by countercurrent chromatography. Thus, for instance, organic substances such as phenols which may occur in low concentration in the water supply may be readily determined by extraction into chloroform.

It is thought that in order for an enzyme to operate in non-aqueous or microaqueous solution a very low concentration of water should be distributed over the surface of the enzyme. Although the role of the water molecules around the enzyme is not fully understood it is believed that the water is necessary for the retention of the enzyme's structure. This places some limitations on the non-aqueous solvents which may be used when carrying out bioelectrochemistry. The solvent should not be so polar that it removes essential water from the enzyme. The solvent will, generally, be organic and hydrophobic solvents such as hydrocarbons are particularly suitable. Other solvents which are more hydrophilic but still water immiscible such as organic halides (of which chloroform is a preferred example), ethers and esters may be used but are preferably saturated with water. Mixtures of any of the above may be used. Non-aqueous solvents which are capable of dissolving enzymes are best avoided.

A further aspect of the present invention is an electrochemical cell for carrying out either of the methods referred to above, the cell comprising an electrode at which an enzyme is retained and containing a non-aqueous or microaqueous solvent.

The electrode utilised in the methods or cell mentioned above may have an enzyme covalently immobilised on it as is conventional in the art. However, the present invention also provides an enzyme electrode for use in a non-aqueous or microaqueous solvent, said electrode comprising a conductor, a hydrophilic support associated with the conductor and an enzyme retained at the support. Preferably, the enzyme is not covalently bound to the support but remains in proximity to it by virtue of the common hydrophilicity of the enzyme and of the support. Thus, the need for conventional enzyme immobilisation eg covalent attachment is avoided. Such electrodes are preferably used in conjunction with microaqueous solvents since the addition of a small quantity of water to the non-aqueous medium ensures retention and stability of the enzyme so that the enzyme electrode may, under suitable conditions, be reused several times.

The conductor with which the hydrophilic support is associated may, for example, be provided by a graphite block or might be on a microstructured electrode (for examples of which see Murray et al (1987)).

The hydrophilic support may be a membrane of a polymeric compound which contains polar residues. The polymeric compound should be one which remains stable in the organic solvent in conjunction with which the enzyme electrode is to be used. Possible polymers include nitrocellulose, cellulose acetate, polyacrylamide and nylon. Nylon is a preferred material.

Alternatively the hydrophilic support may be an inorganic membrane with polar groups at its surface which is thus hydrophilic. For example anodised aluminium membranes such as those sold under the trade name Anopore by Anotech Separations Ltd are suitable. Such membranes may be associated with a conductor to form an enzyme electrode. Another possibility is that a hydrophilic support may be formed on the surface of the conductor e.g where the conductor is aluminium and is provided with an anodised surface.

Another aspect of the present invention is an electrochemical cell comprising an enzyme electrode for use in an organic or microaqueous solvent said electrode comprising a conductor hydrophilic support associated with the conductor and an enzyme retained at said support.

In certain embodiments of the invention a polyphenol oxidase enzyme immobilised at an electrode is employed to detect a phenol as analyte. However, other possible enzymes and analytes may be envisaged.

### BRIEF DESCRIPTION OF THE DRAWINGS

An embodiment of the enzyme electrode of the present invention and of its use will now be exemplified with reference to the accompanying drawings of which
Fig. 1a and 1b respectively show the construction of an electrode and the shape of wire required for electrode construction.
Fig. 2 shows an electrochemical cell with the enzyme electrode of Fig. 1 in place.
Fig. 3 is the calibration curve of the enzyme electrode for p-cresol; and
Fig. 4 shows the electrode response to p-cresol (100µM) over sixteen consecutive assays.

### BEST MODE OF CARRYING OUT THE INVENTION

In the description which follows the enzyme polyphenol oxidase is employed to effect the oxidation of phenols in chloroform solution. Phenols may be partitioned from a large volume of water into a small volume of organic solvent, where they can be rapidly detected through reaction with an enzyme electrode. This provides a method for the determination of low concentrations of phenol in water.

### ELECTRODE CONSTRUCTION

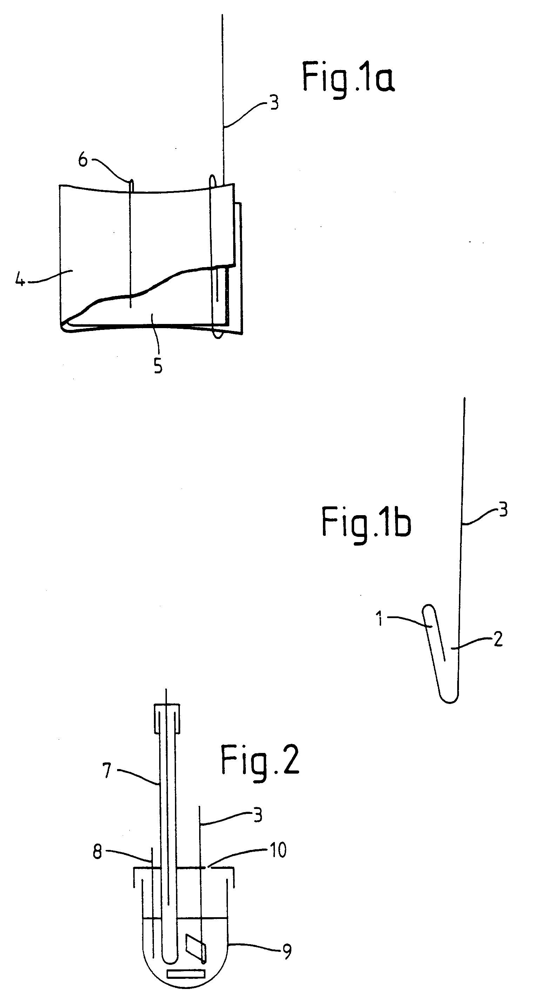

Polyphenol oxidase (1.7 mg tyrosinase from Sigma, Poole, Dorset GB) was dissolved in sodium phosphate buffer (15 µl, 50 mM, pH 7.0). This solution was allowed to soak into a rectangle 4(5x14 mm) of 'Hybond-N' nylon membrane (Amersham International plc, Little Chalfont, Bucks GB.) and left to dry for 1 h at room temperature. The membrane 4 is shown partly cut away in Fig. 1a. A length of bare nickel-chromium wire 3 was folded as in Fig. 1b. One end of the dry nylon membrane 4 was clamped into fold 1 of the length of wire 3. The membrane 4 was then folded around a block of graphite foil 5(5x6x1 mm, Le Carbone, Portslade, Sussex (GB)) which had been soaking in a solution of tetrabutylammonium toluene-4-sulfonate (TBATS) (0.1 M, Fluka, Fluorochem Ltd., Glossop, Derbyshire (GB)) in HPLC grade chloroform for at least 1 h. All chloroform used in this work had been previously saturated with sodium phosphate buffer (50 mM, pH 7.0). One of the shorter edges of the graphite foil block 5 and the unclamped end of the nylon membrane 4 were clamped into fold 2 of the wire. A length (10 mm) of nickel-chromium wire 6 was clamped around the graphite block 5 and membrane 4 to hold the membrane 4 in close contact with the graphite 5. The enzyme electrode is shown in Figure 1a.

### ELECTROCHEMICAL CELL CONSTRUCTION

An electrochemical cell which includes the enzyme electrode of Fig. 1a is shown at Fig. 2. A three electrode system was employed for all work with the enzyme electrode. The potential was maintained by a precision potentiostat (Ministat, Thompson and Associates, Newcastle upon Tyne (GB).) and the current was recorded on an x-t chart recorder (Gallenkamp, Loughborough, Leicestershire (GB).) via a resistance board (J.J. Junior, J.J. Instruments, Southampton, Herts (GB).) A capacitor (47 µF) was connected across the input terminals of the chart recorder to smooth any background noise. The potentiostat, chart recorder, resistance board and capacitor are not shown Fig. 2.

A saturated calomel electrode 7 (Russel pH Ltd., Auchtermuchty, Fife, Scotland) was used as a reference and the auxiliary electrode 8 was a platinum wire (0.4 mm diameter). The electrodes were immersed in chloroform (5 ml, 0.1 M TBATS) contained in a truncated boiling tube 9. The enzyme electrode was poised at -275 mV versus saturated calomel electrode 7 in chloroform and additions of small volumes of stock p-cresol (90 mM) in chloroform (0.1M TBATS) were made via a small hole 10 in the lid of the electrochemical cell.

### CALIBRATION OF THE ENZYME ELECTRODE

Nine discrete assays were performed over a range of p-cresol concentrations (0 to 267 µM) on five different electrodes.

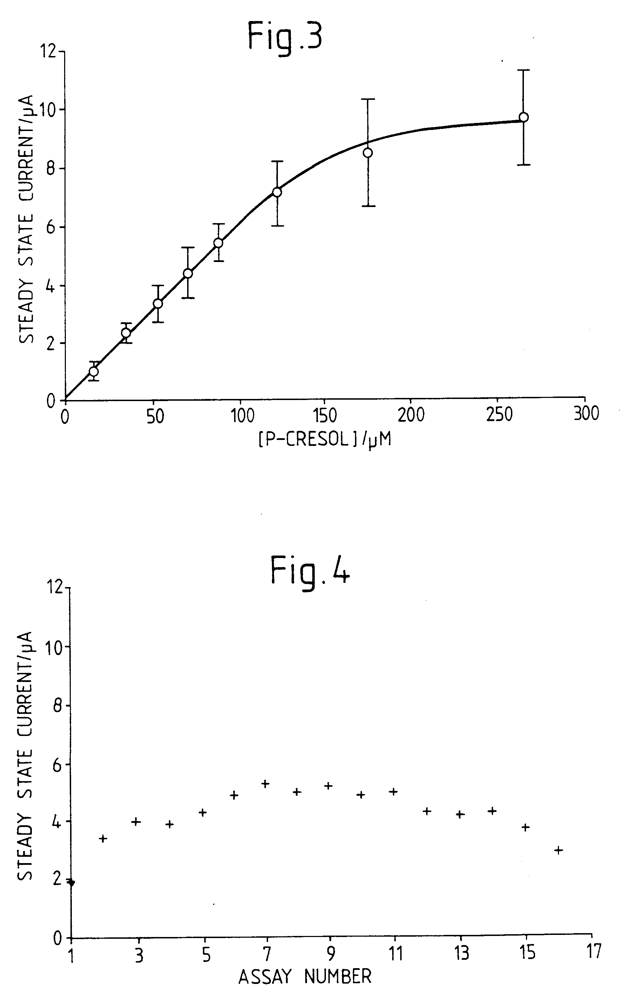

Before each assay sodium phosphate buffer (2µl, 50mM, pH 7.0) was placed onto each side of the enzyme electrode to rehydrate the polyphenol oxidase. The electrode was placed into the cell described above and poised at -275 mV vs standard calomel electrode 7 in chloroform. After 25 minutes the current became constant and an addition of p-cresol was made. An increase in current was then observed which reached a steady value, typically after 3 to 5 minutes. The cell was stirred throughout each assay. After each assay the electrode was removed from the cell and washed in chloroform for about 60 secs before being dried in air prior to the next assay. The response of the electrode to p-cresol was linear in the concentration range 0-100 µM (Figure 3). The standard error bars represent the good reproducibility between electrodes.

### OPERATIONAL STABILITY

The assay procedure outlined above was repeated with a final p-cresol concentration of 100µM for a series of sixteen assays. The response of the electrode increased from 1.9 µA to 4.0 µA in the first three assays and then remained stable over the next eleven assays before starting to fall after assay number fourteen (Figure 4).

### STORAGE STABILITY

Eight electrodes were constructed with dry graphite blocks and their response to p-cresol (200 µM) was recorded. Half were then stored at room temperature and half at 5°C. At each temperature two electrodes were stored dry in bottles containing silica gel, and two were stored in chloroform. Their response to p-cresol (200 µM) was tested again after a few days and then after 7 weeks. The electrodes stored at room temperature showed an average loss of activity of 30% of the response after three days while those stored at 5°C showed no significant decrease in their response to p-cresol (200 µM) (Table 1).

**TABLE 1**

| Storage temp. (°C) | Storage method | Initial response (µA) | Response after 3 days (µA) | Response after 48 days (µA) |
|---|---|---|---|---|
| Room temp. (20°C) | Dry | 5.2 | 6.8 | 3.6 |
| | | 4.8 | 5.4 | 2.8 |
| | In CHCl₃ | 4.0 | 7.0 | 5.5 |
| | | 4.2 | 7.2 | 6.5 |
| 5°C | Dry | 4.3 | 4.4 | 4.7 |
| | | 3.7 | 4.5 | 3.5 |
| | In CHCl₃ | 4.8 | 8.1 | 10.0 |
| | | 3.8 | 9.5 | 7.6 |

### ELECTRODE SPECIFICITY

The response of a single electrode to a standard concentration (100 µM) of phenol, catechol, 4-methyl catechol, m- and p-hydroxy benzaldehyde, m-, p- and o-cresol, p-aminophenol and 4-chlorophenol was recorded. The electrode responded to all the phenols tested except o-cresol and p- and m-hydroxy-benzaldehyde (Table 2), indicating a potential use of the electrode as a phenol sensor.

**TABLE 2**

| Response of the enzyme electrode to ten phenols (100µM) | |
|---|---|
| Phenol | Electrode response (µA) |
| p-cresol | 5.6 |
| m-cresol | 4.7 |
| o-cresol | 0.0 |
| phenol | 6.4 |
| catechol | 8.6 |
| 4-methylcatechol | 6.7 |
| p-hydroxy-benzaldehyde | 0.0 |
| m-hydroxy-benzaldehyde | 0.0 |
| p-aminophenol | 2.4 |
| 4-chlorophenol | 3.1 |

### REFERENCES

1. Yamane, T., Kojima, Y., Ichiryu, T. and Shimizu, S. (1988) Biocatalysis in microaqueous organic solvents. In Enzyme Engineering 9, Annals of the New York Academy of Science.
2. Turner, A.P.F., and Pickup, J.C. (1985), Biosensors 1, 85.
3. Cass, A.E.G., Davis G., Francis, G.D., Hill, H.A.O., Ashton, W.J., Higgins, I.J., Plotkin, E.V., Scott, L.D.L. and Turner, A.P.F. (1984), Anal. Chem., 56, 667-71.
4. Turner, A.P.F., Karube, I. and Wilson, G.S. (1987) Biosensors, Fundamentals and Applications. Oxford University Press.
5. Klibanov, (June 1986) Chemtech, p354.
6. Halling, P.J. (1987) Biotechnology Advances, 5; 47.
7. Kazandijan, R.Z. and Klibanov, A.M. (1985) Journal of the American Chemical Society, 107, 5448.
8. Murray, R.W., Ewing, A.G., and Durst R.A., (1987) Anal. Chem., 59, 379A.

## Claims

1. An enzyme electrode for use in a non-aqueous or microaqueous solvent said electrode comprising a conductor (5), a hydrophilic support (4) associated with the conductor (5), and an enzyme retained at said support (4), characterised in that the arrangement is such that on insertion of the electrode into a said solvent, said solvent contacts the enzyme and a bioelectrochemical reaction catalyzed by said enzyme is effectable in the solvent.

2. An enzyme electrode according to claim 1 wherein the enzyme is retained at the support (4) by their common hydrophilicity and not by covalent binding.

3. An enzyme electrode according to claim 1 wherein the enzyme is present as a component of a whole cell, cell membrane, or organelle.

4. An enzyme electrode according to claim 1 or 2 wherein said support (4) is a membrane of a polymeric compound which contains polar residues or an inorganic membrane having polar groups at its surface.

5. An enzyme electrode according to claim 3 wherein said membrane is of nylon.

6. An enzyme electrode according to claim 1 wherein the immobilized enzyme is a polyphenol oxidase.

7. A method for carrying out a bioelectrochemical reaction in a non-aqueous or microaqueous solvent said method comprising contacting a non-aqueous or microaqueous solution of a substrate for an enzyme with an electrode at which said enzyme is retained and allowing said substrate to undergo reaction at the electrode under the influence of said enzyme; characterised in that said solution contacts the enzyme.

8. A method for the determination of an analyte in a non-aqueous or microaqueous solvent said method comprising; including a non-aqueous or microaqueous solution to be analyzed for said analyte in an electrochemical cell, said cell having an electrode at which an enzyme is retained; and measuring an electrical reponse of said cell; said response arising from a reaction involving said enzyme and analyte and being relatable to the concentration of said analyte; characterised in that said non-aqueous or microaqueous solution contacts said enzyme.

9. A method according to claim 8 wherein the electrode is an enzyme electrode of claim 1.

10. A method according to claim 8 wherein the analyte is a substrate or cofactor of said enzyme, a substance convertible to a substrate or cofactor, of said enzyme, a redox species capable of mediating electron transfer with the enzyme of said electrode or a substance convertible to a redox species capable of mediating electron transfer with the enzyme of said electrode.

11. A method according to claim 9 wherein the analyte is a phenol and the enzyme is a polyphenol oxidase.

12. A method according to claim 6 or claim 7 wherein the solvent is an organic solvent.

13. A method according to claim 6 or claim 7 wherein the solvent is substantially immiscible with water.

14. A method according to claim 6 or claim 7 wherein the solvent is selected from hydrocarbons, organic halides, ethers, esters and mixtures thereof.

15. A method according to any one of claim 6 or claim 7 wherein the solvent is saturated with water.

16. An electrochemical cell comprising an electrode at which an enzyme is retained and containing a non-aqueous or microaqueous solvent whereby a bioelectrochemical reaction is effectable in the solvent, catalysed by said enzyme, characterised in that said non-aqueous or microaqueous solvent is in contact with the enzyme.

17. An electrochemical cell according to claim 16 wherein the electrode is an enzyme electrode comprising a conductor (5), a hydrophilic support (4) associated with the conductor (5), and an enzyme retained at said support (4), the arrangement being such that on insertion of the electrode into a said solvent, said solvent contacts the enzyme.

## Patentansprüche

1. Enzymelektrode zur Verwendung in einem nichtwässerigen oder mikrowässerigen Lösungsmittel, wobei die genannte Elektrode einen Leiter (5), einen mit dem Leiter (5) assoziierten hydrophilen Träger (4) und ein am genannten Träger (4) gehaltenes Enzym umfaßt, dadurch gekennzeichnet, daß die Anordnung so ist, daß beim Einbringen der Elektrode in ein genanntes Lösungsmittel das genannte Lösungsmittel mit dem Enzym in Kontakt steht und eine durch das genannte Enzym katalysierte bioelektrochemische Reaktion im Lösungsmittel bewirkbar ist.

2. Enzymelektrode nach Anspruch 1, worin das Enzym am Träger (4) durch ihre gemeinsame Hydrophilität und nicht durch kovalente Bindung gehalten wird.

3. Enzymelektrode nach Anspruch 1, worin das Enzym als ein Bestandteil einer ganzen Zelle, Zellmembran oder Organelle vorhanden ist.

4. Enzymelektrode nach Anspruch 1 oder 2, worin der genannte Träger (4) eine Membran aus einer Polymerverbindung, die polare Reste enthält, oder eine anorganische Membran mit polaren Gruppen an ihrer Oberfläche ist.

5. Enzymelektrode nach Anspruch 3, worin die genannte Membran aus Nylon besteht.

6. Enzymelektrode nach Anspruch 1, worin das immobilisierte Enzym eine Polyphenoloxidase ist.

7. Verfahren zur Durchführung einer bioelektrochemischen Reaktion in einem nichtwässerigen oder mikrowässerigen Lösungsmittel, wobei das genannte Verfahren das In-Kontakt-Bringen einer nichtwässerigen oder mikrowässerigen Lösung eines Substrats für ein Enzym mit einer Elektrode umfaßt, an der das genannte Enzym gehalten wird, sowie das Unterziehenlassen des genannten Substrats einer Reaktion an der Elektrode unter dem Einfluß des genannten Enzyms; dadurch gekennzeichnet, daß die genannte Lösung mit dem Enzym in Kontakt steht.

8. Verfahren zur Bestimmung eines Analyten in einem nichtwässerigen oder mikrowässerigen Lösungsmittel, wobei das genannte Verfahren umfaßt: das Einbringen einer auf den genannten Analyten hin zu analysierenden nichtwässerigen oder mikrowässerigen Lösung in eine elektrochemische Zelle, wobei die genannte Zelle eine Elektrode aufweist, an der ein Enzym gehalten wird; und das Messen eines elektrischen Ansprechens der genannten Zelle; wobei das genannte Ansprechen aufgrund einer Reaktion entsteht, an der das/der genannte Enzym und Analyt beteiligt sind und die mit der Konzentration des genannten Analyten in Beziehung setzbar ist, dadurch gekennzeichnet, daß die genannte nichtwässerige oder mikrowässerige Lösung mit dem genannten Enzym in Kontakt steht.

9. Verfahren nach Anspruch 8, worin die Elektrode eine Enzymelektrode nach Anspruch 1 ist.

10. Verfahren nach Anspruch 8, worin der Analyt ein Substrat oder Kofaktor des genannten Enzyms ist, eine in ein Substrat oder einen Kofaktor des genannten Enzyms umwandelbare Substanz, eine Redoxspezies, die zur Vermittlung des Elektronentransfers mit dem Enzym der genannten Elektrode fähig ist, oder eine Substanz, die in eine Redoxspezies umwandelbar ist, die zur Vermittlung des Elektronentransfers mit dem Enzym der genannten Elektrode fähig ist.

11. Verfahren nach Anspruch 9, worin der Analyt ein Phenol ist und das Enzym eine Polyphenoloxidase ist.

12. Verfahren nach Anspruch 6 oder 7, worin das Lösungsmittel ein organisches Lösungsmittel ist.

13. Verfahren nach Anspruch 6 oder 7, worin das Lösungsmittel im wesentlichen mit Wasser nicht mischbar ist.

14. Verfahren nach Anspruch 6 oder 7, worin das Lösungsmittel aus Kohlenwasserstoffen, organischen Haliden, Äthern, Estern und Mischungen daraus ausgewählt ist.

15. Verfahren nch einem der Ansprüche 6 oder 7, worin das Lösungsmittel mit Wasser gesättigt ist.

16. Elektrochemische Zelle, die eine Elektrode umfaßt, an der ein Enzym gehalten wird, und ein nichtwässeriges oder mikrowässeriges Lösungsmittel enthält, wodurch im Lösungsmittel eine durch das genannte Enzym katalysierte bioelektrochemische Reaktion bewirkbar ist, dadurch gekennzeichnet, daß das genannte nichtwässerige oder mikrowässerige Lösungsmittel mit dem Enzym in Kontakt steht.

17. Elektrochemische Zelle nach Anspruch 16, worin die Elektrode eine Enzymelektrode ist, die einen Leiter (5), einen mit dem Leiter (5) assoziierten hydrophilen Träger (4) und ein am genannten Träger (4) gehaltenes Enzym umfaßt, wobei die Anordnung so ist, daß beim Einbringen der Elektrode in das genannte Lösungsmittel das genannte Lösungsmittel mit dem Enyzm in Kontakt steht.

## Revendications

1. Electrode d'enzyme à utiliser dans un solvant non aqueux ou micro-aqueux, ladite électrode comprenant un conducteur (5), un support hydrophile (4) associé au conducteur (5) et une enzyme retenue sur ledit support (4), caractérisée en ce que l'arrangement est tel qu'à l'insertion de l'électrode dans un solvant, ledit solvant contacte l'enzyme et qu'une réaction bio-électrochimique catalysée par ladite enzyme puisse être effectuée dans le solvant.

2. Electrode d'enzyme selon la revendication 1, où l'enzyme est retenue sur le support (4) par leur hydrophilicité commune et non pas par liaison covalente.

3. Electrode d'enzyme selon la revendication 1, où l'enzyme est présente en tant que composant d'une cellule entière, d'une membrane de cellule ou d'un organite.

4. Electrode d'enzyme selon la revendication 1 ou 2, où ledit support (4) est une membrane d'un composé polymérique qui contient des résidus polaires ou une membrane inorganique ayant des groupes polaires à sa surface.

5. Electrode d'enzyme selon la revendication 3, où ladite membrane est en nylon.

6. Electrode d'enzyme selon la revendication 1, où l'enzyme immobilisée est une polyphénol oxydase.

7. Méthode pour la mise en oeuvre d'une réaction bio-électrochimique dans un solvant non aqueux ou micro-aqueux, ladite méthode consistant à mettre en contact une solution non aqueuse ou micro-aqueuse d'un substrat pour une enzyme avec une électrode où ladite enzyme est retenue et à permettre audit substrat de subir une réaction à l'électrode sous l'influence de ladite enzyme ; caractérisée en ce que ladite solution contacte l'enzyme.

8. Méthode pour la détermination d'un analyte dans un solvant non aqueux ou micro-aqueux, ladite méthode consistant à introduire une solution non aqueuse ou micro-aqueuse à analyser pour ledit analyte dans une cellule électrochimique, ladite cellule ayant une électrode où est retenue une enzyme; et à mesurer une réponse électrique de ladite cellule ; ladite réponse provenant d'une réaction impliquant ladite enzyme et l'analyte et pouvant être mise en rapport avec la concentration dudit analyte ; caractérisée en ce que ladite solution non aqueuse ou micro-aqueuse contacte ladite enzyme.

9. Méthode selon la revendication 8, où l'électrode est une électrode d'enzyme selon la revendication 1.

10. méthode selon la revendication 8, où l'analyte est un substrat ou cofacteur de ladite enzyme, une substance pouvant être convertie en un substrat ou cofacteur de ladite enzyme, une espèce rédox capable de former le médiateur du transfert d'électrons avec l'enzyme de ladite électrode ou une substance pouvant être convertie en une espèce rédox capable de faire le médiateur du transfert d'électrons avec l'enzyme de ladite électrode.

11. Méthode selon la revendication 9, où l'analyte est un phénol et l'enzyme est une polyphénol oxydase.

12. Méthode selon la revendication 6 ou la revendication 7, où le solvant est un solvant organique.

13. Méthode selon la revendication 6 ou la revendication 7, où le solvant est sensiblement miscible avec l'eau

14. Méthode selon la revendication 6 ou la revendication 7, où le solvant est choisi parmi des hydrocarbures, des halogénures organiques, des éthers, des esters et leurs mélanges.

15. Méthode selon l'une quelconque des revendications 6 ou 7, où le solvant est saturé d'eau.

16. Cellule électrochimique comprenant une électrode où est contenue une enzyme et contenant un solvant non aqueux ou micro-aqueux pour qu'ainsi une réaction bioélèctrochimique puisse être effectuée dans le solvant, catalysée par ladite enzyme, caractérisée en ce que ledit solvant non aqueux ou micro-aqueux est en contact avec l'enzyme.

17. Cellule électrochimique selon la revendication 16, où l'électrode est une électrode d'enzyme comprenant un conducteur (5), un support hydrophile (4) associé au conducteur (5) et une enzyme retenue sur ledit support (4), l'arrangement étant tel qu'à l'insertion de l'électrode dans ledit solvant, le solvant contacte l'enzyme.
